# EUROPÄISCHE PATENTSCHRIFT

(11) **EP 1 467 983 B1**
(45) Veröffentlichungstag und Bekanntmachung des Hinweises auf die Patenterteilung: **05.04.2006**
(21) Anmeldenummer: 03729427.9
(22) Anmeldetag: 07.01.2003
(51) Int. Cl.: C07D 339/04

(54) **VERFAHREN ZUR HERSTELLUNG REINER THIOCTSÄURE**
METHOD FOR PRODUCING THIOCTIC ACID
PROCEDE DE PRODUCTION D'ACIDES THIOCTIQUES PURS

(30) Priorität: 16.01.2002 DE 10201464
(43) Veröffentlichungstag der Anmeldung: 20.10.2004
(73) Patentinhaber: VIATRIS GmbH & Co. KG, 61352 Bad Homburg (DE)
(72) Erfinder: LABAN, Gunter, 01465 Dresden-Langebrück (DE); MEISEL, Peter, 01097 Dresden (DE); MÜLLER, Gilbert, 60596 Frankfurt am Main (DE)
(86) Internationale Anmeldenummer: PCT/EP2003/000064
(87) Internationale Veröffentlichungsnummer: WO 2003/059902

(56) Entgegenhaltungen:
- DE-A- 19 533 881
- DE-C- 19 601 787
- US-A- 4 797 298
- BULMAN PAGE PC: "An enatioselective synthesis of R-(+)-alpha lipoic acid" JOURNAL OF CHEMICAL SOCIETY PERKIN TRANSACTIONS I, 1990, Seite 1615-1618 XP009010605 USA in der Anmeldung erwähnt
- GOPALAN AS ET AL: "Baker's yeast reduction of alkyl 6-chloro-3-oxohexanoates: synthesis of (R)-(+)-alpha lipoic acid" JOURNAL OF CHEMICAL SOCIETY PERKIN TRANSACTIONS I, 1990, Seite 18971900 XP009010601 USA in der Anmeldung erwähnt
- BROOKES MH ET AL: "Synthesis of alpha-(R)- and alpha-(S)-lipoic acid from (S)-malic acid" JOURNAL OF CHEMICAL SOCIETY PERKIN TRANSACTIONS I, 1988, XP009010600 USA in der Anmeldung erwähnt
- DHAR P ET AL: "Piperidinium tetrathiothungstate as sulfur transfer reagent: synthesis of cyclic disulfides" JOURNAL OF ORGANIC CHEMISTRY, Bd. 57, 1992, Seiten 1699-1702, XP002241606 USA
- CRUMP DR: "Synthesis of (2S)-2-propylthietan" AUSTRALIAN JOURNAL OF CHEMISTRY, Bd. 35, 1982, Seiten 1945-1948, XP002241607 australia

## Beschreibung

Die Erfindung betrifft ein Verfahren zur Herstellung von Thioctsäuren der allgemeinen Formel III in Form des Racemats sowie der R- und S-Enantiomere und deren Gemische in hoher Ausbeute und Reinheit.

Wenn im folgenden von Thioctsäure die Rede ist, sind immer sowohl das racemische Gemisch (R,S-Thioctsäure) als auch die enantiomerenreinen Verbindungen (R- und S-Thioctsäure) sowie Mischungen mit beliebigen Gehalten an Enantiomeren zu verstehen.

Thioctsäure ist pharmakologisch wirksam und weist antiphlogistische, antinociceptive sowie zytoprotektive Eigenschaften auf (EP 0427247).

Eine wichtige medizinische Indikation der racemischen Thioctsäure ist die hochdosierte Langzeittherapie der diabetischen Polyneuropathie.

Thioctsäure kann auch Bedeutung bei der Bekämpfung von durch HIV-1- und HTLV III B-Viren bedingter Krankheiten erlangen (A. Baur et al., Klin. Wochenschr. 1991, 69, 722; J.P. Merin et al., FEBS Lett. 1996, 394, 9).

Das R-Enantiomer der Thioctsäure ist ein Naturstoff, der in geringen Konzentrationen in praktisch allen tierischen und pflanzlichen Zellen vorkommt. Die R-Thioctsäure ist als Coenzym bei der oxidativen Decarboxylierung von α-Ketocarbonsäuren (z.B. Brenztraubensäure) von essentieller Bedeutung.

Bei den reinen optischen Isomeren der Thioctsäure ist das R-Enantiomer vorwiegend antiphlogistisch und das S-Enantiomer vorwiegend antinociceptiv wirksam (EP 0427247). Unterschiedliche pharmakokinetische Eigenschaften der beiden Enantiomere sind ebenfalls festgestellt worden (z.B. R. Hermann et al., Eur. J. Pharmaceut. Sci. 1996, 4, 167). Es ist daher sowohl die Synthese des Racemats als auch die der reinen Enantiomere von großer Wichtigkeit.

Ein bekanntes Herstellungsprinzip für die Thioctsäuren der allgemeinen Formel lll besteht in der Reaktion von 6,8-disubstituierten Octansäuren bzw. deren Alkalisalzen und Estern der allgemeinen Formel 1, worin X, Y, R und R' die unten angegebene Bedeutung besitzen und X, Y gleich oder verschieden sein können, mit einem aus äquimolaren Mengen Natriumsulfid und Schwefel hergestelltem Schwefelungsreagenz, das auf Grund seiner stöchiometrischen Zusammensetzung häufig als Natriumdisulfid (Na₂S₂) bezeichnet wird, bzw. in der gemeinsamen Einwirkung von Natriumsulfid (Na₂S) und Schwefel auf die Verbindungen der allgemeinen Formel I (D.S. Acker und W.J. Wayne, J. Am. Chem. Soc. 1957, 79, 6483; A.V. Rama Rao et al., Synth. Commun. 1987, 17, 1339; M.H. Brookes et al., J. Chem. Soc. Perkin Trans. I 1988, 9; P.C.B. Page et al., J. Chem. Soc. Perkin Trans I 1990, 1615; A.S. Gopalan et al., J. Chem. Soc. Perkin Trans. I 1990, 1897; J.S. Yadav et al., J. Carbohydrate Chem., 1990, 9, 307; DE 19533881; G. Bringmann et al., Z. Naturforsch. 54b, 655 (1999)).

Die Verbindungen der allgemeinen Formel I können dabei sowohl als Racemat als auch in Form der R- sowie S-Enantiomere oder deren Gemische eingesetzt werden. Dabei wird so vorgegangen, daß die Verbindungen der allgemeinen Formel I zu einer Lösung des Schwefelungsreagenzes in einem Lösungsmittel wie Ethanol oder DMF hinzugefügt werden. Im Falle der Säuren der allgemeinen Formel I, R=H, erfolgt die gemeinsame Zugabe von Natriumsulfid und Schwefel zum Alkalisalz der Säure.

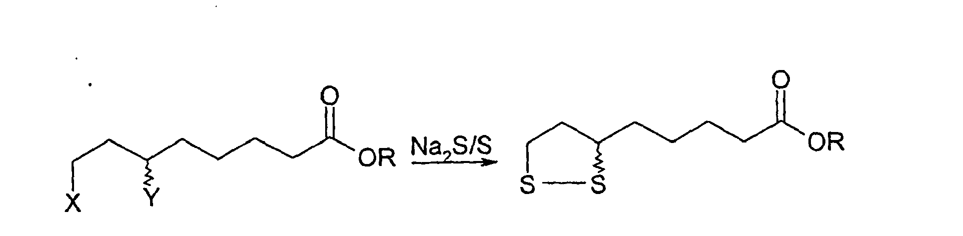

| | |
|---|---|
| I | II R = C₁₋₄ -Alkyl |
| X, Y = Cl, Br, OSO₂R' | III R = H |
| R' = C₁₋₄ -Alkyl, Aryl | |
| R = H, K, Na, C₁₋₄ -Alkyl | |

Die im Falle von R = C₁₋₄ -Alkyl zunächst erhaltenen Ester II werden anschließend durch Zusatz von Alkali im homogenen Medium zur Thioctsäure III hydrolysiert. Sowohl bei der Schwefeleinführung zu II als auch bei der Hydrolyse zu III sind lange Reaktionszeiten erforderlich (z.B. A.V. Rama Rao et al., Synth. Commun. 1987, 17, 1339 : 24 h).

Die unter den angegebenen Bedingungen erhaltenen Thioctsäuren III sind jedoch durch die Verbindungen IV und V, die in Form der Racemate oder als R- bzw. S-Enantiomere vorliegen können, im Bereich von mehreren Prozent verunreinigt.

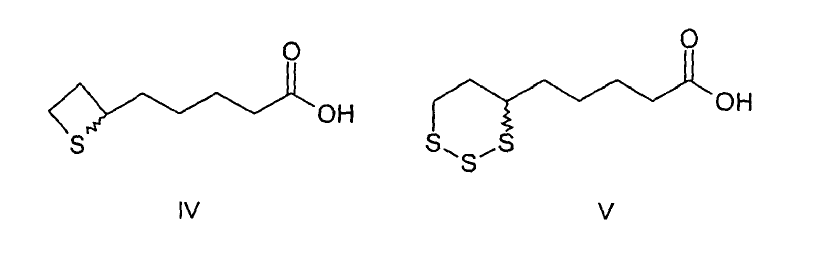

Da die Verbindungen IV und V sich strukturell von der Thioctsäure lediglich nur durch die Anzahl der Schwefelatome unterscheiden, sind die für wirtschaftliche Trenn- und Reinigungsoperationen wie Destillation und Umkristallisation erforderlichen Unterschiede der physikochemischen Eigenschaften, wie z.B. Flüchtigkeit und Löslichkeit, nur gering. Die nachträgliche Abtrennung der während der Reaktion gebildeten Nebenprodukte IV und V ist extrem aufwendig und somit auf wirtschaftliche Weise nicht möglich.Eine Detektion ist nur durch spezielle analytische Methoden, wie z.B. HPLC, möglich. Daß bei den bisher bekannten Verfahren auch die Verbindungen IV und V gebildet wurden, ist nicht verwunderlich, denn es ist allgemein bekannt, daß bei der Umsetzung von Natriumsulfid (Na₂S) mit Schwefel ein Gemisch von Sulfiden (Na₂Sₓ) unterschiedlicher Kettenlänge x entsteht. Dies führt dazu, daß bei der Umsetzung von 6,8-disubstituierten Octansäuren sowie deren Alkalisalzen und Estern mit Natriumsulfid / Schwefel (Molverhältnis 1:1) nicht nur die auf Grund der Stöchiometrie erwartete Thioctsäure gebildet wird (x = 2, Einbau einer S₂-Einheit), sondern durch Einbau von "S₁" (wenn x = 1) die 5-(2-Thiacyclobutyl)-valeriansäure IV sowie von "S₃" (wenn x = 3) die 5-(1,2,3)Trithian-4-yl-pentansäure V neben höheren aliphatischen und cyclischen Polysulfiden gebildet werden.

Deshalb wurden die mit der einstufigen Direkteinführung des Schwefels in 6,8-disubstituierte Octansäuren sowie deren Alkalisalze und Ester der allgemeinen Formel verbundenen Qualitätsprobleme häufig dadurch umgangen, indem die rohe Thioctsäure zunächst durch NaBH₄ zur 6,8-Dimercapto-octansäure reduziert wurde, die in einer weiteren Stufe zur Thioctsäure oxidiert werden mußte (z.B. DE 19533881).

Dadurch wird jedoch nur die Abreicherung der reduktiv zur 6,8-Dimercapto-octansäure abbaubaren Verbindung V erreicht, nicht jedoch die Entfernung von IV.

Noch aufwendiger ist die Schwefeleinführung über die Umsetzung der 6,8-disubstituierten Octansäuren und deren Ester mit Thiohamstoff zu den Salzen der 6,8-Bis(amidiniumthio)-octansäure, deren Spaltung zur 6,8-Dimercapto-octansäure und Oxidation zur Thioctsäure (3-Stufen-Verfahren, z.B. F. Balkenhohl und J. Paust, Z. Naturforsch. 54b, 649 (1999); DE 19601787).

Zusammenfassend kann festgestellt werden, daß die beschriebene Arbeitsweise mangelhaft ist und ein wirtschaftliches Verfahren zur Herstellung reiner Thioctsäure der allgemeinen Formel III nicht existiert. Die Bereitstellung von reiner Thioctsäure ist jedoch auch auf Grund der o.g. hochdosierten Langzeittherapie besonders wichtig.

Aufgabe der Erfindung ist es deshalb, ein wirtschaftliches Herstellungsverfahren für die Verbindung III in hoher Reinheit und Ausbeute bereitzustellen.

Diese Aufgabe wird dadurch gelöst, daß man zuerst ein Sulfid der allgemeinen Formel VI,

M₂S VI

wobei M ein Alkali- oder Ammoniumion bedeutet, zu einem Gemisch, bestehend aus suspendiertem Schwefel und der Lösung einer racemischen, (R)- oder (S)-6,8-disubstituierten Octansäure, der entsprechenden Alkalisalze sowie Alkylester der allgemeinen Formel I, zudosiert und anschließend ein Sulfit der allgemeinen Formel VII,

M'₂SO₃ VII

wobei M' ein Alkali-, Ammonium- oder ein halbes Erdalkaliion bedeutet, einwirken läßt. Es ist dabei auch möglich, Gemische der Verbindungen der allgemeinen Formel I einzusetzen. Das Sulfid VI kann dabei in fester Form oder in wäßriger oder wäßrig-alkoholischer Lösung zudosiert werden. Die wässrig-alkoholische Lösung kann ein Gemisch aus Wasser und einem niederen Alkohol mit 1 bis 3 C-Atomen sein. Das Sulfit VII kann als festes Salz oder in wäßriger Lösung zugegeben werden.

Dabei ist es im Falle der Ester der allgemeinen Formel I, R = C 1-4 -Alkyl, auch möglich, das Sulfit der allgemeinen Formel VII zu Beginn oder während der alkalischen Hydrolyse von II zu III zuzugeben. Weiterhin kann das Sulfit der allgemeinen Formel VII auch jeweils in Teilmengen nach der Zugabe des Sulfides der allgemeinen Formel VI sowie zu Beginn oder während der alkalischen Hydrolyse von II zu lll hinzugefügt werden.

Als inerte, mit Wasser mischbare Lösungsmittel für die Verbindungen der allgemeinen Formel eignen sich sowohl polare, protische Lösungsmittel, wie niedere Alkohole mit 1 bis 3 C-Atomen als auch dipolar-aprotische Lösungsmittel wie beispielsweise Dimethylformamid, N-Methylpyrrolidon oder Aceton sowie unpolare Lösungsmittel wie beispielsweise Toluol oder n-Heptan. Die angegebenen Lösungsmittel können auch als Gemische eingesetzt werden.

Es ist auch möglich, Mischungen von Wasser sowohl mit den reinen Lösungsmitteln als auch mit den Lösungsmittelgemischen zu verwenden.

Das Molverhältnis 1 : VI : Schwefel : VII beträgt dabei 1 : 1 : 1 : 0.5 bis 1 : 1.5 : 2 : 3, vorzugsweise
1 : 1.1 : 1.5 : 1 bis 1 : 1,1 : 1,5 : 2

Die Schwefeleinführung kann bei Temperaturen von 0 bis 100°C, vorzugsweise im Bereich von 5 bis 90 °C,durchgeführt werden.

In speziellen Fällen, wie beispielsweise beim Einsatz der (S)- sowie (R)-8-Chlor-6-sulfonyloxy-octansäuren, der entsprechenden Alkalisalze sowie Alkylester der allgemeinen Formel (X = Cl, Br, Y = OSO₂R') ist es notwendig, zur Vermeidung von Racemisierungen die Reaktionstemperatur abzustufen, indem die Sulfid-Zugabe bei 30 - 50 °C, vorzugsweise 35 - 45 °C, durchgeführt wird und erst danach die Reaktionstemperatur erhöht wird.

Die alkalische Hydrolyse von II zu III erfolgt schonend im Zweiphasensystem Cyclohexan bzw. Methyl-tert.butylether / verdünnte Alkalilauge bei Temperaturen von 30 bis 90 °C, üblicherweise mit Reaktionszeiten < 5 h.

Die Reaktionszeiten sowohl bei der Schwefeleinführung zu II als auch bei der Hydrolyse zu III sind gegenüber dem Stand der Technik wesentlich verkürzt.

Die Thioctsäuren III werden in Abhängigkeit vom eingesetzten Octansäurederivat I in Ausbeuten bis über 80 % in sehr reiner Form ohne die Verbindungen IV und V erhalten.

In einer besonderen Ausführungsform der Erfindung ist es auch möglich, bei ansonsten erfindungsgemäßer Arbeitsweise auf die Zugabe des Sulfits der allgemeinen Formel VII während der Umsetzung von I zu II bzw. zu III zu verzichten und die insbesonders stark mit der Verbindung V verunreinigten Rohprodukte zunächst zu isolieren.

Die Reinprodukte lassen sich durch Einwirkung von Sulfiten der allgemeinen Formel VII, gegebenenfalls in Gegenwart von inerten Lösungsmitteln wie Cyclohexan oder Methyl-tert.butylether, auf die in verdünnten Alkalilaugen gelösten Rohprodukte erhalten.

Im folgenden wird die Erfindung anhand von Beispielen näher erläutert, ohne sie jedoch einzuschränken.

### Beispiel 1

Man gibt eine Lösung von 4,0 g (0,1 Mol) Natriumhydroxid in 10 ml Wasser zu 100 ml Ethanol (96 %) und fügt nacheinander 21,3 g (0,1 Mol) rac. 6,8-Dichloroctansäure (D.S. Acker und W.J. Wayne, J. Amer Chem. Soc. 1957, 79, 6483) und 4,8 g (0,15 Mol) Schwefel unter Rühren hinzu. Das Gemisch wird zum leichten Rückfluß erhitzt (Innentemperatur 78 - 80 °C). Unter Rühren tropft man während ca.2 h bei leichtem Rückfluß eine Lösung von 13,8 g (0,11 Mol) Natriumsulfid-Hydrat (Gehalt: 62 % Na₂S), gelöst in einem Gemisch von 70 ml Wasser und 40 ml Ethanol (96 %), zu.

Anschließend läßt man eine Lösung von 18,9 g (0,15 Mol) Natriumsulfit in 75 ml Wasser zulaufen und rührt 1 h bei einer Innentemperatur von ca. 80 °C nach. Man kühlt auf ca. 50 °C ab und fügt 600 ml Wasser sowie 250 ml eines Gemisches Cyclohexan / Ethylacetat 4 : 1 hinzu. Bei 35 - 40 °C wird unter Rühren mit 10 %iger Salzsäure angesäuert (pH 1). Man trennt
die Phasen, extrahiert 2 x mit je 100 ml Cyclohexan / Ethylacetat 4 : 1 bei 35 - 40 °C nach und engt die vereinigten organischen Phasen im Vakuum schonend (Badtemperatur bis 40° C) ein. Man kühlt unter Rühren auf 0 bis 5 °C (Beginn der Kristallisation) und anschließend 2 bis 3 h bei -5 bis -10 °C. Nach dem Waschen mit kaltem Cyclohexan und Trocknen (30 °C) erhält man 17,7 bis 18,4 g (86 - 89 % d.Th.) reine R,S-Thioctsäure.
Fp 61 °C (aus Cyclohexan /Ethylacetat 4:1)

### Beispiel 2

Man fügt unter Rühren 24,1g (0,1 Mol) 6,8-Dichloroctansäureethylester und anschließend 4,8 g (0,15 Mol) Schwefel zu einem Gemisch von 50 ml Ethanol und 50 ml n-Propanol.

Nach dem Erwärmen auf eine Innentemperatur von 82 bis 84°C (Rückfluß) tropft man unter intensivem Rühren während 2 bis 2,5 h eine Lösung von 13,8 g (0,11 Mol) Natriumsulfid-Hydrat (Gehalt:62 % Na₂S) in 80 ml Wasser und 40 ml Ethanol zu.

Dann gibt man eine Lösung von 18,9 g (0,15 Mol) Natriumsulfit in 75 ml Wasser zügig zu und erhitzt noch 1 h bei leichtem Rückfluß.Man kühlt auf etwa 50°C ab, fügt 200 ml Cyclohexan und 300 ml Wasser hinzu, stellt bei einer Innentemperatur von 30 bis 35°C unter Rühren mit 10%iger Salzsäure auf einen pH-Wert von ca. 1 und rührt ca. 15 min nach. Man trennt die Phasen, fügt die Cyclohexan-Phase zu einer Lösung von 8 g (0,2 Mol) Natriumhydroxid in 600 ml Wasser und rührt das Zweiphasensystem ca. 4h intensiv bei einer Innentemperatur von ca. 70°C (leichter Rückfluß).

Man kühlt auf ca. 50°C ab, trennt die Phasen, fügt zur wäßrigen Phase 500 ml Cyclohexan/Ethylacetat (95:5) hinzu und säuert bei einer Innentemperatur von 35 bis 40°C unter Rühren mit
ca. 10%iger Salzsäure an (pH 1 bis 2). Nach der Phasentrennung extrahiert man mit weiteren 200 ml Cyclohexan/Ethylacetat (95:5) nach.

Die vereinigten organischen Phasen werden mit 1g Diacel 300 BL (Filtrierhilfsmittel) ca. 10 min bei ca. 30°C gerührt und nach dem Filtrieren im Vakuum bei maximal 40°C Badtemperatur auf ca. 200 ml eingeengt. Man läßt unter Rühren bei -5 bis -10°C 4 bis 6 h kristallisieren. Nach dem Waschen mit kaltem Cyclohexan und Trocknen bei 30°C erhält man 14,6 bis 15,7 g (71 bis 76% d.Th.) reine R,S-Thioctsäure.
Fp 61 °C (aus Cyclohexan/Ethylacetat)

### Beispiel 3

Zu einer Lösung von 22,7 g (0,1 Mol) (S)-6,8-Dichloroctansäuremethylester (DE 19533881) in einem Gemisch von 50 ml Ethanol und 50 ml n-Propanol fügt man unter Rühren 4,8 g (0,15 Mol) Schwefel hinzu.

Man erwärmt auf eine Innentemperatur von 82 bis 84 °C (Rückfluß) und tropft unter intensivem Rühren während ca. 2 h eine Lösung von 13,8 g (0,11 Mol) Natriumsulfid-Hydrat (Gehalt: 62 % Na₂S in 80 ml Wasser und 40 ml Ethanolzu. Dann gibt man eine Lösung von 18,9 g (0,15 Mol) Natriumsulfit in 75 ml Wasser zügig zu, rührt noch 1 h bei leichtem Rückfluß nach, kühlt auf 40 - 50 °C ab, fügt 200 ml Methyl-tert.butylether (MTBE) und 300 ml Wasser zu, säuert bei einer Innentemperatur von 30 - 35 °C mit 10 %iger Salzsäure an (pH 1-2) und rührt 15 min nach. Man trennt die Phasen, fügt die MTBE-Phase zu einer Lösung von 8 g (0,2 Mol) Ätznatron in 600 ml Wasser und rührt das Zweiphasensystem 2 - 3 h intensiv bei einer Innentemperatur von ca. 55 °C (Rückfluß). Man kühlt auf 40 - 50 °C ab, trennt die Phasen und fügt zur wäßrigen Phase 300 ml Cyclohexan hinzu.

Bei 35 - 40 °C wird unter Rühren mit 10 %iger Salzsäure angesäuert (pH 1-2). Man trennt die Phasen, extrahiert mit 300 ml Cyclohexan bei 35 - 40 °C nach, rührt die vereinigten Cyclohexan-Phasen 10 min mit 1 g Diacel 300 BL (Filtrierhilfsmittel) bei 35 bis 40°C aus, filtriert und engt im Vacuum bei maximal 35 °C auf ca. 30% des ursprünglichen Volumens ein. Man rührt 2 bis 3 h bei 6 - 10 °C, wäscht das Kristallisat mit kaltem Cyclohexan (2 x 5 ml) und trocknet bei Raumtemperatur.
Ausbeute: 15,2 bis 16,3 g (74 - 79 % d. Th.) reine R-Thioctsäure.
Fp 49 - 50 °C (aus Cyclohexan)

### Beispiel 4

Eine Lösung von 28,7 g (0,1 Mol) (S)-8-Chlor-6-mesyloxy-octansäuremethylester (DE 19533881) in Toluol (66 %ige Lösung, 43,5 g) gibt man zu 50 ml Ethanol und 50 ml n-Propanol und fügt unter Rühren 4,8 g (0,15 Mol) Schwefel hinzu,
erwärmt auf eine Innentemperatur von 40 - 42 °C und tropft unter intensivem Rühren während 1 h eine Lösung von 13,8 g (0,11 Mol) Natriumsulfid-Hydrat (Gehalt: 62 % Na₂S) in 80 ml Wasser und 40 ml Ethanolzu. Man
erwärmt zum Rückfluß (80 bis 84°C), tropft während 1 h eine Lösung von 18,9 g (0,15 Mol) Natriumsulfit in 75 ml Wasser zu und rührt 1 h unter Rückfluß nach.Anschließend kühlt man auf 40 - 50 °C ab, fügt 200 ml Cyclohexan und 300 ml Wasser zu, säuert bei einer Innentemperatur von 30 - 35 °C mit 10 %iger Salzsäure an (pH 1-2) und rührt 15 min nach.

Man trennt die Phasen, fügt die Cyclohexan-Phase zu einer Lösung von 8 g (0,2 Mol) Ätznatron
in 600 ml Wasser hinzu und rührt das Zweiphasensystem 2 - 3 h intensiv bei einer Innentemperatur von ca. 60 °C. Man kühlt auf 40 - 50 °C ab, trennt die Phasen und gibt zur wäßrigen Phase 300 ml Cyclohexan.

Bei 35 - 40 °C wird unter Rühren mit 10 %iger Salzsäure angesäuert (pH 1-2). Man trennt die Phasen, extrahiert mit 300 ml Cyclohexan bei 35 - 40 °C nach, rührt die vereinigten Cyclohexan-Phasen 10 min mit 1 g Diacel 300 BL (Filtrierhilfsmittel) aus, filtriert und engt im Vakuum bis zu einer Badtemperatur von 40°C auf ca. 30% des ursprünglichen Volumens ein. Man rührt 2 bis 3 h bei 5 - 10 °C, wäscht das Kristallisat mit kaltem Cyclohexan (2 x 5 ml) und trocknet bei Raumtemperatur.
Ausbeute: 15,2 bis 16,3 g (-74 bis 79 % d. Th.) reine R-Thioctsäure.
Fp 49 - 50 °C (aus Cyclohexan)

### Beispiel 5

Zu einer Lösung von 22,7 g (0,1 Mol) (S)-6,8-Dichloroctansäuremethylester (DE 19533881) in einem Gemisch von 50 ml Ethanol und 50 ml n-Propanol fügt man unter Rühren 4,8 g (0,15 Mol) Schwefel hinzu.

Man erwärmt auf eine Innentemperatur von 82 bis 84 °C (Rückfluß) und tropft unter intensivem Rühren während ca. 2 h eine Lösung von 13,8 g (0,11 Mol) Natriumsulfid-Hydrat (Gehalt:62% Na₂S) in 80 ml Wasser und 40 ml Ethanol zu. Dann rührt man noch 1 h bei leichtem Rückfluß nach, kühlt auf 40 bis 50°C ab, fügt 200 ml Cyclohexan und 300 ml Wasser zu , säuert bei einer Innentemperatur von 30 bis 35°C mit 10%iger Salzsäure an (pH 1 -2) und rührt 15 min nach. Man trennt die Phasen, fügt die Cyclohexan-Phase zu einer Lösung von 8 g (0,2 Mol) Natriumhydroxid und 18,9 g (0,15Mol) Natriumsulfit in 600 ml Wasser und rührt das Zweiphasensystem 2 h intensiv bei einer Innentemperatur von ca. 60°C. Man kühlt auf 40 - 50°C ab, trennt die Phasen und fügt zur wäßrigen Phase 300 ml Cyclohexan hinzu.

Bei 35 - 40°C wird unter Rühren mit 10%iger Salzsäure angesäuert (pH 1-2). Man trennt die Phasen, extrahiert mit 300 ml Cyclohexan bei 35 - 40°C nach, rührt die vereinigten Cyclohexan-Phasen 10 min mit 1 g Diacel 300 BL (Filtrierhilfsmittel) bei 35 - 40°C aus, filtriert und engt im Vakuum bei maximal 40°C Badtemperatur auf ca. 30% des ursprünglichen Volumens ein. Man rührt 2 - 3 h bei 5 - 10°C, wäscht das Kristallisat mit kaltem Cyclohexan (2 x 5 ml) und trocknet bei Raumtemperatur.
Ausbeute: 14,4 bis 15,2 g (70 bis 74% d.Th.) reine R-Thioctsäure
Fp 49 - 50°C (aus Cyclohexan)

### Beispiel 6

Zu einer Lösung von 22,7 g (0,1 Mol) (S)-6,8-Dichloroctansäuremethylester (DE 19533881) in einem Gemisch von 50 ml Ethanol und 50 ml n-Propanol fügt man unter Rühren 4,8 g (0,15 Mol) Schwefel hinzu. Man erwärmt auf eine Innentemperatur von 82 bis 84 °C (Rückfluß), tropft unter intensivem Rühren während ca. 2 h eine Lösung von 13,8 g (0,11 Mol)

Natriumsulfid-Hydrat (Gehalt: 62% Na₂S) in 80 ml Wasser und 40 ml Ethanol zu und fügt eine
Lösung von 9,5 g (0,075 Mol) Natriumsulfit in 40 ml Wasser hinzu. Dann rührt man noch 1 h bei
leichtem Rückfluß nach, kühlt auf 40 bis 50°C ab, fügt 200 ml Cyclohexan und 300 ml Wasser zu und säuert bei einer Innentemperatur von 30 bis 35°C mit 10%iger Salzsäure an (pH 1-2). Man trennt die Phasen, fügt die Cyclohexan-Phase zu einer Lösung von 8 g (0,2 Mol) Natriumhydroxid und 9,5 g (0,075) Natriumsulfit in 600 ml Wasser und rührt das Zweiphasensystem 2 h intensiv bei einer Innentemperatur von 68 - 70°C (Rückfluß).
Man kühlt auf 40 - 50°C ab,trennt die Phasen und fügt zur wäßrigen Phase 300 ml Cyclohexan hinzu. Bei 35 - 40°C wird unter Rühren mit 10%iger Salzsäure angesäuert (pH 1-2). Man trennt die Phasen, extrahiert mit 300 ml Cyclohexan bei 35 - 40°C nach, rührt die vereinigten Cyclohexan-Phasen 10 min mit 1 g Diacel 300 BL (Filtrierhilfsmittel) bei 35 - 40°C aus, filtriert und engt im Vakuum bei maximal 40°C Badtemperatur auf ca. 30% des usprünglichen Volumens ein. Man rührt 2 - 3 h bei 6 - 10°C, wäscht das Kristallisat mit kaltem Cyclohexan (2 x 5 ml) und trocknet bei Raumtemperatur.

Ausbeute: 15,0 bis 16,1 g (73 bis 78% d.Th.) reine R-Thioctsäure

### Beispiel 7

### Reinigung von roher Thioctsäure

5,1 g rohe R-Thioctsäure (Gehalt V: 14%) und 4 g Natriumsulfit wurden in verdünnte Natronlauge (1 g NaOH gelöst in 150 ml Wasser) eingetragen. Die Lösung wurde 3 h bei einer Innentemperatur von 60 - 62°C gerührt. Nach dem Abkühlen wurden 75 ml Cyclohexan hinzugefügt und bei 35 - 40°C unter Rühren mit verdünnter Salzsäure sauer gestellt (pH 1 -2). Nach der Phasentrennung wurde mit 75 ml Cyclohexan bei 35 bis 40°C nachextrahiert.

Die vereinigten Extrakte wurden im Vakuum bei einer maximalen Badtemperatur von 40°C auf ca. 1/4 des Volumens eingeengt. Die Lösung wurde 2 -3 h bei 5 -10°C gerührt, das Kristallisat mit kaltem Cyclohexan gewaschen und bei Raumtemperatur getrocknet.
Ausbeute: 4,1 g reine R-Thioctsäure
Fp 50 bis 51°C

## Patentansprüche

1. Verfahren zur Herstellung von racemischer, R- oder S-Thioctsäure oder deren Gemische der allgemeinen Formel III, **dadurch gekennzeichnet, dass** man die Lösung eines Sulfides der allgemeinen Formel VI zu einem Gemisch, bestehend aus suspendiertem Schwefel und der Lösung einer racemischen, (R)- oder (S)-6,8-disubstituierten Octansäure, der entsprechenden Alkalisalze, Alkylester sowie Gemischen der allgemeinen Formel l, worin X, Y, R und R' die unten angegebene Bedeutung besitzen, zudosiert und anschließend , gegebenenfalls während oder nach der Hydrolyse eines intermediär gebildeten Esters II, ein Sulfit der allgemeinen Formel VII, einwirken läßt. 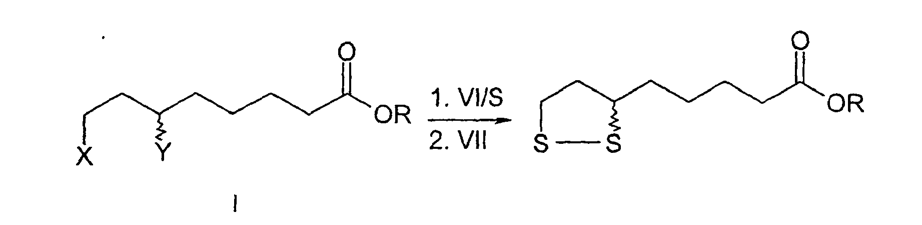
| | |
|---|---|
| X, Y = Cl, Br, OSO₂R' | II R = C₁₋₄ -Alkyl |
| R' = C₁₋₄-Alkyl, Aryl | III R = H |
| R = H, K, Na, C₁₋₄ -Alkyl | |
| M₂S | M'₂SO₃ |
| VI | VII |
| M = K, Na, NH₄ | M' = K, Na, NH₄, Mg/2, Ca/2, Ba/2 |

2. Verfahren nach Anspruch 1, **dadurch gekennzeichnet, dass** das Molverhältnis I : VI : Schwefel : VII dabei 1 : 1 : 1 : 0.5 bis 1 : 1.5 : 2 : 3, vorzugsweise 1 : 1.1 : 1.5 : 1 bis 1 : 1,1 : 1,5: 2 beträgt.

3. Verfahren nach Anspruch 1, **dadurch gekennzeichnet, dass** bei Einsatz der racemischen, (R)- oder (S)-6,8-disubstituierten Octansäureester oder deren Gemische der allgemeinen Formel I (R = C₁₋₄ -Alkyl) der zunächst gebildete racemische, R- oder S-Thioctsäureester der allgemeinen Formel II zu racemischer, R- oder S-Thioctsäure oder deren Gemischen der allgemeinen Formel III hydrolysiert wird.

4. Verfahren nach Anspruch 1, **dadurch gekennzeichnet, dass** das Sulfid der allgemeinen Formel VI in Form einer wässrigen oder wässrig-alkoholischen Lösung zudosiert wird.

5. Verfahren nach Anspruch 1, **dadurch gekennzeichnet, dass** als Lösungsmittel für die Verbindungen der allgemeinen Formel I polare protische Lösungsmittel, dipolar aprotische Lösungsmittel, unpolare Lösungsmittel, Gemische der genannten Lösungsmittel sowie Mischungen mit Wasser verwendet werden.

6. Verfahren nach Anspruch 1, **dadurch gekennzeichnet, dass** das Sulfit der allgemeinen Formel VII als festes Salz oder in Form einer wäßrigen Lösung zugegeben wird.

7. Verfahren zur Hydrolyse roher racemischer, R-oder S-Thioctsäureester der allgemeinen Formel II im Zweiphasensystem zu reiner racemischer, R-oder S-Thioctsäure oder deren Gemischen in Gegenwart von Sulfiten der allgemeinen Formel VI.

8. Verfahren zur Reinigung von racemischer, R-oder S-Thioctsäure oder deren Gemische durch Einwirkung von Sulfiten der allgemeinen Formel VI, gegebenenfalls in Gegenwart von inerten Lösungsmitteln, auf die in verdünnten Alkalilaugen gelösten Rohprodukte.

## Claims

1. Method for producing racemic, R- or S-thioctic acid or mixtures thereof of the general formula III, **characterized in that** the solution of a sulphide of the general formula VI is metered into a mixture consisting of suspended sulphur and a solution of a racemic, (R)- or (S)-6,8-disubstituted octanoic acid, the corresponding alkali metal salts, alkyl esters and mixtures of the general formula I, in which X, Y, R and R' have the meanings stated below, and then, optionally during or after the hydrolysis of an ester II formed as an intermediate, a sulphite of the general formula VII is allowed to act. 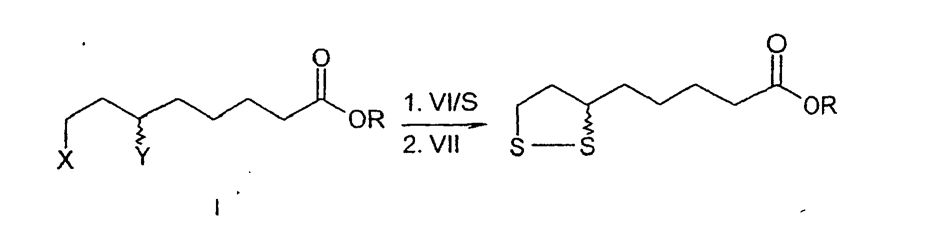
| | |
|---|---|
| X, Y = Cl, Br, OSO₂R' | II R = C₁₋₄-alkyl |
| R' = C₁₋₄-alkyl, aryl | III R = H |
| R = H, K, Na, C₁₋₄-alkyl | |
| M₂S | M'₂SO₃ |
| VI | VII |
| M =K, Na, NH₄ | M' = K, Na, NH₄, Mg/2, Ca/2, Ba/2 |

2. Method according to Claim 1, **characterized in that** the molar ratio I : VI : sulphur : VII is 1 : 1 : 1 : 0.5 to 1 : 1.5 : 2 : 3, preferably 1 : 1.1 : 1.5 : 1 to 1 : 1.1 : 1.5 : 2.

3. Method according to Claim 1, **characterized in that**, with the use of the racemic, (R)- or (S)-6,8-disubstituted octanoic esters or mixtures thereof of the general formula I (R = C₁₋₄-alkyl), the initially formed racemic, R- or S-thioctic ester of the general formula II is hydrolysed to give racemic, R- or S-thioctic acid or mixtures thereof of the general formula III.

4. Method according to Claim 1, **characterized in that** the sulphide of the general formula VI is metered in in the form of an aqueous or aqueous alcoholic solution.

5. Method according to Claim 1, **characterized in that** polar protic solvents, dipolar aprotic solvents, nonpolar solvents, mixtures of said solvents and mixtures with water are used as solvents for the compounds of the general formula I.

6. Method according to Claim 1, **characterized in that** the sulphite of the general formula VII is added as a solid salt or in the form of an aqueous solution.

7. Method for hydrolysing crude racemic, R- or S-thioctic esters of the general formula II in the two-phase system to give pure racemic R- or S-thioctic acid or mixtures thereof in the presence of sulphites of the general formula VI.

8. Method for purifying racemic, R- or S-thioctic acid or mixtures thereof by the action of sulphites of the general formula VI, optionally in the presence of inert solvents, on the crude products dissolved in dilute alkali solutions.

## Revendications

1. Procédé de fabrication d'acide R- ou S-thioctique racémique ou de leurs mélanges de formule générale III, **caractérisé en ce que** l'on ajoute de manière dosée la solution d'un sulfure de formule générale VI à un mélange constitué de soufre en suspension et de la solution d'un acide octanoïque racémique (R)- ou (S)-disubstitué en 6,8, de ses sels alcalins correspondants, de ses esters alkyliques ainsi que de mélanges de formule générale I, où X, Y, R et R' possèdent la signification indiquée ci-dessous, et **en ce qu'**on fait ensuite agir, éventuellement pendant ou après l'hydrolyse d'un ester II formé de manière intermédiaire, un sulfite de formule générale VII : 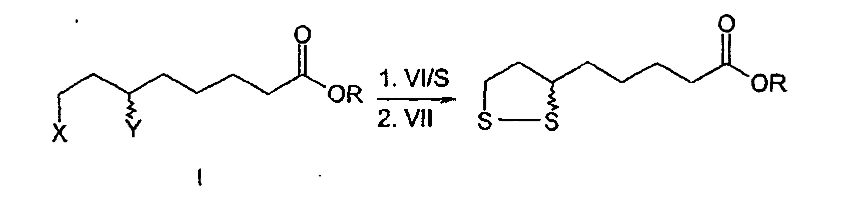
| | |
|---|---|
| X, Y = Cl, Br, OSO₂R' | II R = alkyle en C₁-C₄ |
| R' = alkyle en C₁-C₄, aryle | III R = H |
| R = H, K, Na, alkyle en C₁-C₄ | |
| M₂S | M'₂SO₃ |
| VI | VII |
| M = K, Na, NH₄ | M' = K, Na, NH₄, Mg/2, Ca/2, Ba/2 |

2. Procédé selon la revendication 1, **caractérisé en ce que** le rapport molaire I:VI:soufre:VII se situe en l'occurrence dans la plage de 1:1:1:0,5 à 1:1,5:2:3, de préférence de 1:1,1:1,5:1 à 1:1,1:1,5:2.

3. Procédé selon la revendication 1, **caractérisé en ce que**, lors de l'utilisation des esters d'acide octanoïque racémique (R)- ou (S)-disubstitué en 6,8 ou de leurs mélanges de formule générale I (R = alkyle en C₁ à C₄); on hydrolyse l'ester d'acide R- ou S-thioctique racémique d'abord formé de formule générale II pour former de l'acide racémique R- ou S-thioctique ou ses mélanges de formule générale III.

4. Procédé selon la revendication 1, **caractérisé en ce que** le sulfure de formule générale VI est ajouté de manière dosée sous la forme d'une solution aqueuse ou aqueuse-alcoolique.

5. Procédé selon la revendication 1, **caractérisé en ce que** l'on utilise comme solvants pour les composants de formule générale I des solvants polaires protiques, des solvants dipolaires aprotiques, des solvants apolaires, des mélanges des solvants cités ainsi que des mélanges avec de l'eau.

6. Procédé selon la revendication 1, **caractérisé en ce que** le sulfite de formule générale VII est ajouté comme sel solide ou sous la forme d'une solution aqueuse.

7. Procédé d'hydrolyse d'esters bruts racémiques d'acide R- ou S-thioctique de formule générale II dans le système à deux phases pour former un acide pur racémique R- ou S-thioctique ou ses mélanges en présence de sulfites de formule générale VI.

8. Procédé de purification d'acide racémique R- ou S-thioctique ou de ses mélanges par l'action de sulfites de formule générale VI, éventuellement en présence de solvants inertes, sur les produits bruts dissous dans des lessives alcalines diluées.
